# EUROPÄISCHE PATENTSCHRIFT

(11) **EP 0 863 979 B1**
(45) Veröffentlichungstag und Bekanntmachung des Hinweises auf die Patenterteilung: **14.03.2001**
(21) Anmeldenummer: 96931035.8
(22) Anmeldetag: 06.09.1996
(51) Int. Cl.: C11D 11/00, C11D 1/66

(54) **VERFAHREN ZUR HERSTELLUNG WASSER- UND STAUBFREIER ZUCKERTENSIDGRANULATE**
METHOD FOR PREPARING GRANULATED ANHYDROUS AND NON-DUSTING SUGAR SURFACTANTS
PROCEDE DE FABRICATION DE GRANULES TENSIOACTIFS DE SUCRE SANS EAU NI POUSSIERE

(30) Priorität: 15.09.1995 DE 19534371
(43) Veröffentlichungstag der Anmeldung: 16.09.1998
(73) Patentinhaber: Cognis Deutschland GmbH, 40589 Düsseldorf (DE)
(72) Erfinder: LÜDER, Thomas, D-40764 Langenfeld (DE); SCHOLINAKIS, Konstantinos, D-40789 Monheim (DE); HENSEN, Hermann, D-42781 Haan (DE); SEIPEL, Werner, D-40723 Hilden (DE); ROTTMANN, Mirella, D-40231 Düsseldorf (DE)
(86) Internationale Anmeldenummer: EP9603912
(87) Internationale Veröffentlichungsnummer: WO9710324

(56) Entgegenhaltungen:
- EP-A- 0 572 957
- DE-A- 4 209 339
- DE-A- 4 306 757

## Beschreibung

### Gebiet der Erfindung

Die Erfindung betrifft ein Verfahren zur gleichzeitigen Trocknung und Granulierung von wäßrigen Zukkertensidpasten unter Verwendung eines Dünnschichtverdampfers.

### Stand der Technik

Zuckertenside, wie beispielsweise Alkyloligoglucoside oder Fettsäure-N-alkylglucamide, zeichnen sich durch ausgezeichnete Detergenseigenschaften und hohe ökotoxikologische Verträglichkeit aus. Aus diesem Grund gewinnen diese Klassen nichtionischer Tenside in zunehmendem Maße an Bedeutung. Werden sie bislang in der Regel in flüssigen Formulierungen, wie beispielsweise Geschirrspülmittel oder Haarshampoos eingesetzt, so besteht inzwischen auch ein Marktbedürfnis nach festen, wasserfreien Anbietungsformen, die sich beispielsweise auch in Pulverwaschmittel oder Syndetseifen einarbeiten lassen.

Die Trocknung flüssiger Tensidzubereitungen erfolgt großtechnisch in der Regel durch konventionelle Sprühtrocknung, bei der man die wäßrige Tensidpaste am Kopf eines Turmes in Form feiner Tröpfchen versprüht, denen heiße Trocknungsgase entgegengeführt werden. Diese Technologie ist auf Zuckertensidpasten jedoch nicht ohne weiteres anwendbar, da die für die Trocknung erforderlichen Temperaturen oberhalb der Karamelisierungs- d.h. Zersetzungstemperatur der Zuckertenside liegen. Kurz gesagt: Bei konventioneller Trocknung von Zuckertensidpasten werden verkohlte Produkte erhalten, zudem kommt es zu Anbackungen an der Turmwandung, die in kurzen Abständen eine aufwendige Reinigung erforderlich machen.

In der Vergangenheit hat man versucht, dieses Problem zu umgehen. Aus der Deutschen Patentanmeldung **DE-A1 41 02 745** (Henkel) ist z.B. ein Verfahren bekannt, bei dem man Fettalkoholpasten eine geringe Menge von 1 bis 5 Gew.-% Alkylglucosiden zusetzt und einer konventionellen Sprühtrocknung unterwirft. Allerdings läßt sich der Prozeß nur in Gegenwart einer großen Menge anorganischer Salze durchführen. In der Deutschen Patentanmeldung **DE-A1 41 39 551** (Henkel) wird vorgeschlagen, Pasten von Alkylsulfaten und Alkylglucosiden, die jedoch maximal 50 Gew.-% des Zuckertensids enthalten können, in Gegenwart von Mischungen aus Soda und Zeolithen zu versprühen. Hier werden jedoch nur Compounds erhalten, die eine geringe Tensidkonzentration und ein unzureichendes Schüttgewicht aufweisen. Die Patentanmeldung **DE-A1 42 09 339** beschreibt ein Verfahren zur Herstellung von Zuckertensidgranulaten, welches wäßrige APG-Pasten in einen Turbinentrockner mit rotierenden Einbauten gleichzeitig trocknet und in Stücke geformt bringt; der Rest Wassegehalt beträgt 1,5 bis 1,6%. Die europäische Patentanmeldung **EP-A1 572 957** beschreibt einen Dünnschichtverdampfer für die Trocknung anionischer Tenside, die erreichten Schüttdichten sind 490 bis 510 g/l, der Rest Wassergehalt wird mit 1 bis 8% angegeben, über die Komgrößenverteilung wird nichts vermerkt. Schließlich wird in der Internationalen Patentanmeldung **WO 95/14519** (Henkel) darüber berichtet, Zuckertensidpasten einer Trocknung mit überhitztem Wasser-dampf zu unterwerfen; dies ist jedoch technisch sehr aufwendig. Tatsächlich existiert bislang kein zu-verlässiges Verfahrens, das die Herstellung von qualitativ hochwertigen, praktisch wasserfreien Zucker-tensidpulvem bzw. -granulaten gestattet und dabei ohne die Mitverwendung von Trägem während des Trocknungsvorgangs auskommt. Ein weiteres Problem besteht ferner darin, daß Verfahren des Stands der Technik nicht zu den besonders bevorzugten Schwerpulvern mit einem Schüttgewicht oberhalb von 500 g/l mit einem gleichzeitig stark verminderten Staubgehalt führt. Gerade diese beiden Parameter sind jedoch aus wirtschaftlichen, anwendungstechnischen und sicherheitstechnischen Gründen von be-sonderer Bedeutung.

Demzufolge hat die komplexe Aufgabe der vorliegenden Erfindung darin bestanden, wäßrige Zuckertensidpasten mit möglichst geringem technischen Aufwand ohne Mitverwendung von anorganischen oder organischen Trägern in praktisch wasser- und staubfreie Granulate zu überführen, die sich gleichzeitig durch eine akzeptable Farbqualität, ein hohes Schüttgewicht, gute Rieselfähigkeit, eine zufriedenstellende Lagerstabilität und im Vergleich zu den Produkten des Stands der Technik durch zumindest vergleichbare anwendungstechnische Eigenschaften auszeichnen.

### Beschreibung der Erfindung

Gegenstand der Erfindung ist ein Verfahren zur Herstellung von wasser- und staubfreien - d.h. der Anteil an Teilchen mit einem Durchmesser unterhalb von 200 µm liegt unterhalb von 5 Gew.-% - Zuckertensidgranulaten mit hoher Schüttdichte im Bereich von 550 bis 650 g/l und einer mittleren Korngröße von 2,0 bis 2,8 mm, bei dem man wäßrige Pasten von
a) Alkyl- und/oder Alkenyloligoglykosiden und/oder
b) Fettsäure-N-alkylpolyhydroxyalkylamiden
mit einem Feststoffgehalt von mindestens 20 und vorzugsweise im Bereich von 25 bis 75 Gew.-% in einem horizontal angeordneten Dünnschichtverdampfer mit rotierenden Einbauten bis auf einen Restwassergehalt unterhalb von 2, vorzugsweise unter 1,5 und insbesondere unter 1 Gew.-% gleichzeitig trocknet und in stückige Form bringt, wobei man an den Dünnschichtverdampfer vom Produkteingang bis zum Produktaustrag einen Temperaturgradienten anlegt und im Gegenstrom mit Luft begast.

Überraschenderweise wurde gefunden, daß ein horizontal angeordneter Dünnschichtverdampfer in idealer Weise geeignet ist, wäßrige Zuckertensidpasten in trockene, hellfarbige, rieselfähige und nichtklebrige Granulate zu überführen, ohne daß es zu Produktverfärbungen und Anbacken an den Wandungen kommt. Die Produkte weisen eine hohe Schüttdichte im Bereich von 550 bis 650 g/l und einen mittleren Korndurchmesser von 2,0 bis 2,8 mm auf, was zu einer Verminderung der unerwünschten Wasseraufnahme und des Zusammenbackens der Partikel führt. Auf diese Weise ist auch eine hohe Lagerstabilität gegeben. Gleichzeitig sind sie staubfrei, d.h. der Anteil an Teilchen mit einem Durchmesser unterhalb von 200 pm liegt unterhalb von 5 Gew.-%. Diese Erkenntnis ist um so überraschender, da Bauteile der genannten Art zwar grundsätzlich für die Trocknung von Aniontensidpasten bekannt sind, die dabei resultierenden Pulver jedoch weder im Hinblick auf das Schüttgewicht, noch den Staub- und Restwassergehalt die genannten Anforderungen erfüllen.

### Alkyl- und/oder Alkenyloligoglykoside

Alkyl- und Alkenyloligoglykoside stellen bekannte nichtionische Tenside dar, die der Formel (**I**) folgen,

**R**^{**1**}**O-[G]**_{**p**} **(I)**

in der R¹ für einen Alkyl- und/oder Alkenylrest mit 4 bis 22 Kohlenstoffatomen, G für einen Zuckenest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1-0 301 298** und **WO 90/03977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel (**I**) gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkylund/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vor-zugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Fettsäure-N-alkylpolyhydroxyalkylamide

Fettsäure-N-alkylpolyhydroxyalkylamide stellen nichtionische Tenside dar, die der Formel (**II**) folgen, 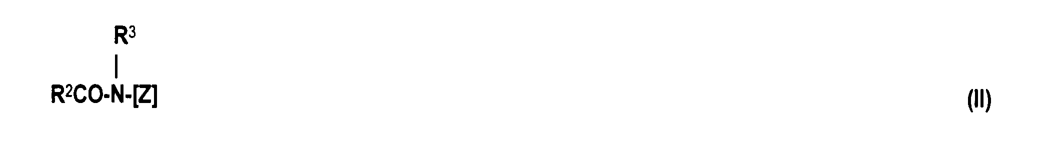 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens. Surf. Det. 25, 8 (1988)**. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel (**III**) wiedergegeben werden:

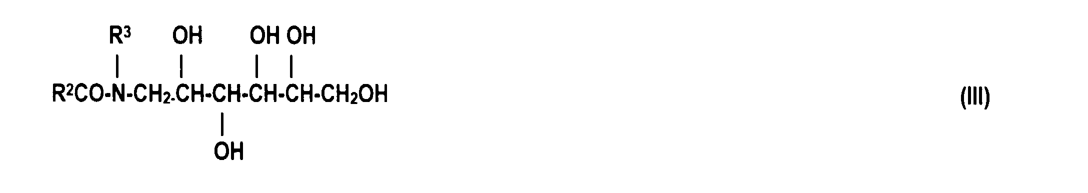

Vorzugsweise werden als Fettsäure-N-alkylpolyhydroxyalkylamide Glucamide der Formel (**III**) eingesetzt, in der R³ für Wasserstoff oder eine Alkylgruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkyl-glucamide der Formel (**III**), die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

### Trocknen und Granulieren im Flash Dryer

Die gleichzeitige Trocknung und Granulierung erfolgt in einem horizontal angeordneten Dünnschichtverdampfer mit rotierenden Einbauten, wie er z.B. von der Firma VRV unter der Bezeichnung "Flash Dryer" vertrieben wird. Hierbei handelt es sich, vereinfacht dargestellt, um ein Rohr, das über mehrere Zonen hinweg unterschiedlich temperiert werden kann. Über eine oder mehrere Wellen, die mit Blättern oder Flugscharen als rotierende Einbauten versehen sind, wird das pastöse Einsatzmaterial, das über eine Pumpe eindosiert wird, gegen die beheizte Wandung geschleudert, an der die Trocknung in einer dünnen Schicht von typischerweise 1 bis 10 mm Stärke erfolgt. Im Sinne der Erfindung hat es sich als vorteilhaft erwiesen, an den Dünnschichtverdampfer einen Temperaturgradienten von 170 (Produkteinlaß) auf 20°C (Produktaustrag) anzulegen. Hierzu können beispielsweise die beiden ersten Zonen des Verdampfers auf 160°C geheizt und die letzte auf 20°C gekühlt werden. Höhere Trocknungstemperaturen haben sich im Hinblick auf die thermische Labilität der Einsatzstoffe als nicht vorteilhaft erwiesen. Der Dünnschichtverdampfer wird bei atmosphärischem Druck betrieben und im Gegenstrom mit Luft (Durchsatz 50 bis 150 m³/h) begast. Die Eintrittstenmperatur des Gases liegt in der Regel bei 20 bis 30, die Austrittstemperatur bei 90 bis 110°C.

Die wäßrigen Zuckertensidpasten, die als Einsatzstoffe in Betracht kommen, können einen Feststoffgehalt im Bereich oberhalb von 20, vorzugsweise von 25 bis 75 Gew.-% aufweisen; typischerweise liegt er bei 30 bis 50 Gew.-%. Die Durchsatzmenge ist natürlich von der Größe des Trockners abhängig, liegt jedoch typischerweise bei 5 bis 15 kg/h. Es empfiehlt sich, die Pasten bei der Einspeisung auf 40 bis 60°C zu temperieren.

Nach der Trocknung hat es sich weiterhin als sehr vorteilhaft erwiesen, die noch etwa 50 bis 70°C heißen Granulate auf ein Förderband, vorzugsweise eine Schwingrinne zu geben und dort rasch, d.h. innerhalb einer Verweilzeit von 20 bis 60 s, mit Umgebungsluft auf Temperaturen von etwa 30 bis 40°C abzukühlen. Zur weiteren Verbesserung der Beständigkeit gegenüber unerwünschter Wasseraufnahme kann man die Granulate auch anschließend durch Zugabe von 0,5 bis 2 Gew.-% Kieselsäure abpudern.

### Gewerbliche Anwendbarkeit

Die nach der erfindungsgemäßen Verfahren erhältlichen Granulaten können anschließend mit weiteren Inhaltsstoffen von pulverförmigen oberflächenaktiven Mitteln, wie beispielsweise Turmpulvern für Waschmittel abgemischt werden. Es ist ferner problemlos möglich, die Pulver in wäßrige Zubereitungen einzuarbeiten. Tatsächlich werden bei Verwendung der Pulver gegenüber den wäßrigen Ausgangspasten keine Unterschiede in den anwendungstechnischen Eigenschaften beobachtet. Auch in Syndetseifenrezepturen lassen sich die Granulate beispielsweise zusammen mit Fettsäuren, Fettsäuresalzen, Stärke, Polyglycolen und dergleichen ohne weiteres einarbeiten.

### Beispiele

### Beispiel 1

Die Herstellung der Granulate erfolgte in einem Flash Dryer der VRV S.p.A., Mailand/lT. Es handelte sich hierbei um einen horizontal angeordneten Dünnschichtverdampfer (Länge 1100 mm, Innendurchmesser : 155 mm) mit 4 Wellen und 22 Blättern, deren Abstand zur Wandung 2 mm betrug. Der Trockner besaß drei separate Heiz- bzw. Kühlzonen und eine Wärmeaustauscherfläche von insgesamt 0,44 m². Der Betrieb erfolgte bei Normaldruck. Über eine Schwingpumpe wurde eine auf 50°C temperierte wäßrige Paste eines Kokosalkyloligoglucosids (Plantaren® APG 1200, Feststoffgehalt ca. 50 Gew.-%) mit einem Durchsatz von 11,5 kg/h in den Dünnschichtverdampfer gepumpt, dessen Heizzonen 1 und 2 auf 160°C und dessen Kühlzone 3 auf 20°C eingestellt worden waren. Die Geschwindigkeit der Rotoren betrug 24 m/s. Der Flash Dryer wurde mit Luft (ca. 110 m³/h) begast; die Gasaustrittstemperatur betrug ca. 65°C. Das vorgetrocknete, noch etwa 60°C heiße Granulat wurde auf eine Schwingrinne (Länge 1 m) gegeben, mit Raumluft begast und innerhalb von 30 s auf etwa 40°C abgekühlt. Anschließend wurde es mit etwa 1 Gew.-% Kieselsäure (Sipemat® 50 S) abgepudert. Es wurde ein trockenes, rein-weißes, auch nach längerer Lagerung an der Luft rieselfähiges und nicht-klumpendes Granulat erhalten, das nach Einarbeitung in Shampoorezepturen keine Unterschiede zu einem pastösen Vergleichsprodukt zeigte. Die Kenndaten des Granulats sind in Tabelle 1 wiedergegeben:

**Tabelle 1**

| **Kenndaten des Flash Dryer Granulats** | |
|---|---|
| **Parameter** | **Granulat** |
| Korngrößenverteilung [Gew.-%] < 1,0 mm | 1,2 |
| 1,0 mm | 47,8 |
| 2,5 mm | 16,5 |
| 3,2 mm | 16,2 |
| 4,0 mm | 11,3 |
| 5,0 mm | 4,7 |
| 6,2 mm | 2,3 |
| Restwassergehalt (nach Fischer) [Gew.-%] | 1,0 |
| Schüttdichte [g/l] | 590 |

### Beispiel 2

Beispiel 1 wurde wiederholt, anstelle des Glucosids jedoch Kokosfettsäure-N-methylglucamid eingesetzt. Die Temperatur in den beiden Heizzonen des Flash Dryers wurde auf 170°C angehoben. Es wurde ebenfalls ein rein weißes, rieselfähiges und nicht-klumpendes Granulat erhalten, das eine Schüttdichte von 600 g/l und einen Restwassergehalt von 0,8 Gew.-% aufwies.

## Patentansprüche

1. Verfahren zur Herstellung von wasser- und staubfreien - d.h der Anteil an Teilchen mit einem Durchmesser unterhalb von 200 µm liegt unterhalb von 5 Gew.-% - Zuckertensidgranulaten mit hoher Schüttdichte im Bereich von 550 bis 650 g/l und einer mittleren Korngröße von 2,0 bis 2,8 m, bei dem man wäßrige Pasten von
a) Alkyl- und/oder Alkenyloligoglykosiden und/oder
b) Fettsäure-N-alkylpolyhydroxyalkylamiden
mit einem Feststoffgehalt von mindestens 20 Gew.-% in einem horizontal angeordneten Dünnschichtverdampfer mit rotierenden Einbauten bis auf einen Restwassergehalt unterhalb von 2 Gew.-% gleichzeitig trocknet und in stückige Form bringt, wobei man an den Dünnschichtverdampfer vom Produkteingang bis zum Produktaustrag einen Temperaturgradienten anlegt und im Gegenstrom mit Luft begast.

2. Verfahren nach Anspruch 1, **dadurch gekennzeichnet**, daß man Alkyl- und Alkenyloligoglykoside der Formel (I) einsetzt,
**R**^{**1**}**O-[G]**_{**p**} **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Verfahren nach den Ansprüchen 1 und 2, **dadurch gekennzeichnet**, daß man Fettsäure-N-alkylpolyhydroxyalkylamide der Formel (**II**) einsetzt, 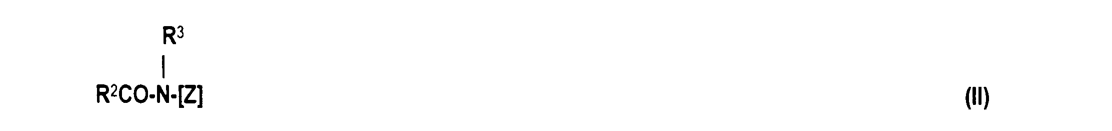 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

4. Verfahren nach den Ansprüchen 1 bis 3, **dadurch gekennzeichnet**, daß mah vom Produkteintrag bis zum -austrag einen Temperaturgradienten von 170 bis auf 20°C anlegt.

5. Verfahren nach den Ansprüchen 1 bis 4, **dadurch gekennzeichnet**, daß man das die noch heißen Granulate nach Ausschleusen aus dem Dünnschichtverdampfer auf einer Schwingrinne mit Umgebungsluft weiter abkühlt.

6. Verfahren nach den Ansprüchen 1 bis 5, **dadurch gekennzeichnet**, daß man die Granulate nach dem Abkühlen mit Kieselsäure abpudert.

## Claims

1. A process for the production of water-free and dust-free, i.e. the percentage content of particles smaller than 200 µm in diameter is below 5% by weight, sugar surfactant granules with a high bulk density of 550 to 650 g/l and a mean particle size of 2.0 to 2.8 mm, in which water-containing pastes of
a) alkyl and/or alkenyl oligoglycosides and/or
b) fatty acid-N-alkyl polyhydroxyalkylamides
with a solids content of at least 20% by weight are dried in a horizontally arranged thin-layer evaporator with rotating internals to a residual water content below 2% by weight and, at the same time, converted into particulate form, a temperature gradient being applied to the thin-layer evaporator from the product entrance to the product exit and air being passed through in countercurrent.

2. A process as claimed in claim 1, **characterized in that** alkyl and alkenyl oligoglycosides corresponding to general formula **(I)**:
**R**^{**1**}**O-[G]**_{**p**} **(I)**
in which R¹ is an alkyl and/or alkenyl group containing 4 to 22 carbon atoms, G is a sugar unit containing 5 or 6 carbon atoms and p is a number of 1 to 10, are used.

3. A process as claimed in claims 1 and 2, **characterized in that** fatty acid N-alkyl polyhydroxyalkylamides corresponding to formula **(II)**: 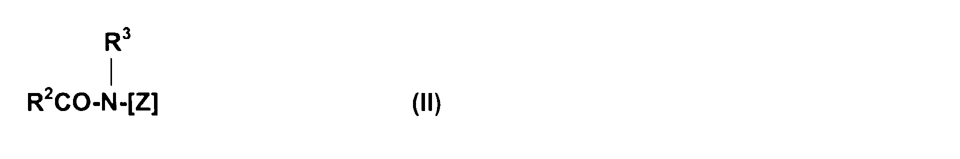 in which R²CO is an aliphatic acyl groupl containing 6 to 22 carbon atoms, R³ is hydrogen, an alkyl or hydroxyalkyl group containing 1 to 4 carbon atoms and [Z] is a linear or branched polyhydroxyalkyl group containing 3 to 12 carbon atoms and 3 to 10 hydroxyl groups, are used.

4. A process as claimed in claims 1 to 3, **characterized in that** a temperature gradient of 170 to 20°C is applied to the thin-layer evaporator from the product entrance to the product exit.

5. A process as claimed in claims 1 to 4, **characterized in that**, after leaving the thin-layer evaporator, the still hot granules are further cooled with ambient air on a vibrating chute.

6. A process as claimed in claims 1 to 5, **characterized in that**, after cooling, the granules are dusted with silica powder.

## Revendications

1. Procédé de production de granulés tensioactifs glucidiques anhydres et dépourvus de poussière - c'est-à-dire ayant une proportion de particules de diamètre inférieur à 200 µm qui se situe en dessous de 5 % en poids - avec une haute densité apparente comprise entre 550 et 650 g/l et une granulométrie moyenne de 2,0 à 2,8 mm, dans lequel on sèche et on transforme en morceaux des pâtes aqueuses de
a) alkyl- et/ou alcényloligoglycosides et/ou
b) N-alkylpolyhydroxyalkylamides d'acides gras
avec des teneurs en solides d'au moins 20 en poids dans un vaporisateur en couche mince disposé horizontalement avec pièces en rotation, jusqu'à une teneur résiduelle en eau inférieure à 2 % en poids, opération dans laquelle on applique au vaporisateur en couche mince, depuis l'entrée jusqu'à la sortie du produit, un gradient de température, et on y fait passer de l'air à contre-courant.

2. Procédé selon la revendication 1,
caractérisé en ce qu'
on utilise des alkyl- et alcényloligoglycosides de formule (I)
R¹O-[G]ₚ (I)
dans laquelle R¹ représente un radical alkyle et/ou alcényle comportant de 4 à 22 atomes de carbone, G représente un radical glucidique comportant 5 ou 6 atomes de carbone et p représente des nombres de 1 à 10.

3. Procédé selon les revendications 1 et 2,
caractérisé en ce qu'
on utilise des N-polyhydroxy alkylamides d'acides gras de formule (II) 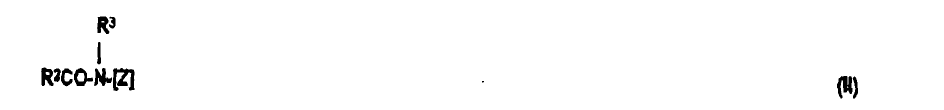 dans laquelle R²CO représente un radical acyle aliphatique comportant de 6 à 22 atomes de carbone, R³ représente un hydrogène, un radical alkyle ou hydroxyalkyle comportant de 1 à 4 atomes de carbone et [Z] représente un radical polyhydroxyalkyle linéaire ou ramifié comportant de 3 à 12 atomes de carbone et de 3 à 10 groupes hydroxyle.

4. Procédé selon les revendications 1 à 3,
caractérisé en ce qu'
on applique de l'entrée à la sortie du produit un gradient de température de 170 à 20°C.

5. Procédé selon les revendications 1 à 4,
caractérisé en ce qu'
on refroidit plus avant les granulés encore chauds après leur sortie de l'évaporateur en couche mince avec de l'air environnant dans un convoyeur oscillant.

6. Procédé selon les revendications 1 à 5,
caractérisé en ce qu'
on débarrasse les granulés de leur poudre avec de l'acide silicique après refroidissement.
